(19) Europäisches Patentamt / European Patent Office / Office européen des brevets

(11) EP 4 442 291 B1

(12) **EUROPEAN PATENT SPECIFICATION**

(45) Date of publication and mention of the grant of the patent:
**19.11.2025 Bulletin 2025/47**

(21) Application number: **22915690.6**

(22) Date of filing: **12.12.2022**

(51) International Patent Classification (IPC):
***A61L 31/06*** *(2006.01)* ***A61M 5/158*** *(2006.01)*
***A61B 5/15*** *(2006.01)* ***A61L 31/04*** *(2006.01)*
***A61L 31/14*** *(2006.01)*

(52) Cooperative Patent Classification (CPC):
(C-Sets available)
**A61M 5/158; A61B 5/15003; A61B 5/150389; A61B 5/150412; A61B 5/150519; A61B 5/150549; A61B 5/150717; A61L 31/048; A61L 31/141**
(Cont.)

(86) International application number:
**PCT/JP2022/045600**

(87) International publication number:
**WO 2023/127466 (06.07.2023 Gazette 2023/27)**

(54) **MEDICAL NEEDLE**

MEDIZINISCHE NADEL

AIGUILLE MÉDICALE

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**

(30) Priority: **27.12.2021 JP 2021211836**

(43) Date of publication of application:
**09.10.2024 Bulletin 2024/41**

(73) Proprietor: **Terumo Kabushiki Kaisha**
**Tokyo 151-0072 (JP)**

(72) Inventor: **TAKEDA, Norihiko**
**Fujinomiya-shi, Shizuoka 418-0004 (JP)**

(74) Representative: **TBK**
**Bavariaring 4-6**
**80336 München (DE)**

(56) References cited:
**WO-A1-2021/029393**
**CN-A- 111 499 988**
**JP-A- S59 118 165**
**JP-A- S59 118 166**
**JP-A- S59 118 166**
**JP-A- S6 210 153**
**US-A- 4 655 764**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).



**(Cont. next page)**

(52) Cooperative Patent Classification (CPC): (Cont.)

C-Sets
**A61L 31/048, C08L 27/06**

## Description

### Technical Field

The present invention relates to a medical needle according to the preamble of claim 1, including a needle body, a hub, and a protector. Such a medical needle is known from JP 1-17381 B.

### Background Art

A blood sampling needle is a type of medical needle. A blood sampling needle is incorporated into, for example, a blood sampling kit for collecting blood from a subject (hereinafter also referred to as "donor"). In this case, the blood sampling needle is connected to a bag through a tube. The blood sampling needle includes a hollow needle body, a hub, and a protector. A proximal portion of the needle body in a longitudinal direction is inserted into the hub. A distal portion of the needle body in the longitudinal direction is exposed from the hub. The distal portion is inserted into a blood vessel of the donor.

The tube is connected to the hub. The proximal portion of the needle body is connected to the tube through the hub. Therefore, the blood collected from the distal portion of the needle body passes through the hollow needle body and flows into the tube. The blood that has passed through the tube flows into the bag.

As described in JP 1-17381 B, before use, a protector of a blood sampling needle is joined to a hub. Therefore, a distal portion exposed from the hub is covered with the protector. In other words, the distal portion is protected by the protector. When collecting blood with the blood sampling needle, a user who collects the blood twists the protector relative to the hub. With this operation, the protector is separated from the hub. The user pulls the separated protector away from the hub. Accordingly, the distal portion of the needle body is exposed from the protector.

Materials of the hub and the protector are both a resin composition. In the resin composition, a plasticizer is added to a base resin. The plasticizer maintains flexibility of the base resin. An example of the base resin includes polyvinyl chloride (PVC). JP 1-17381 B discloses a phthalate or trimellitate as a preferable plasticizer when PVC is used as a base resin.

### Summary of Invention

### Technical Problem

Among phthalates, di-2 ethylhexyl phthalate (hereinafter referred to as "DEHP") has been widely used in the related art. On the other hand, recently, trimellitates have been frequently employed as plasticizers. However, according to intensive studies by the present inventors, properties of a hub and a protector prepared using a trimellitate are different from properties of a hub and a protector prepared using DEHP. In other words, it is difficult to obtain desired properties simply by replacing DEHP with a trimellitate as a plasticizer. This causes a difficulty in separating the protector from the hub. In addition, there is a concern that the needle body is misaligned with the hub.

An object of the present invention is to solve the problems.

### Solution to Problem

According to the present invention there is provided a medical needle according to claim 1 which includes: a needle body; a hub that supports a proximal portion of the needle body in a longitudinal direction; and a protector that protects the needle body, wherein the protector has a needle sheath that covers a portion of the needle body exposed from the hub, the needle sheath is separably joined to the hub, the hub includes a first resin composition and the needle sheath includes a second resin composition, the first resin composition contains polyvinyl chloride and a trimellitate which accounts for 10 to 20 phr, and the second resin composition contains polyvinyl chloride and a trimellitate which accounts for 40 to 50 phr. Preferred embodiments of the present invention are defined in the dependent claims.

According to the present invention, there is provided a protector that has appropriate hardness which enables transmission of rotary torque with ease. Therefore, the protector can be easily separated from a hub. The hub also provides an excellent retaining force with respect to a needle body. This eliminates a concern such that the needle body is misaligned with the hub.

### Brief Description of Drawings

Fig. 1 is a perspective view of a blood sampling needle which is a type of medical needle.
Fig. 2 is a longitudinal cross-sectional view illustrating main parts of the blood sampling needle.
Fig. 3 is a graph illustrating average rotary torque when a protector is separated from a hub.
Fig. 4 is a graph illustrating average tensile strength when a needle body is drawn from the hub.
Fig. 5 is a graph illustrating an average attachment/detachment force when the protector is attached to and detached from the hub.
Fig. 6 illustrates SAXS scattering curves obtained by performing small-angle X-ray scattering (SAXS) on Sample A and Sample B.
Fig. 7 is a chart illustrating a result of curve fitting performed on the SAXS scattering curve of Sample A.
Fig. 8 is a schematic view of an ideal crystal.
Fig. 9 is a schematic view of amorphousness.
Figs. 10A and 10B are schematic views of a tissue of Sample A.
Fig. 11 is a chart illustrating a result of curve fitting performed on the SAXS scattering curve of Sample B.
Figs. 12A and 12B are schematic views of a tissue of Sample B.
Fig. 13 illustrates WAXS scattering curves obtained by performing wide-angle X-ray scattering (WAXS) on Sample A and Sample B.
Fig. 14 is a chart illustrating peaks separated for analysis on the WAXS scattering curve of Sample A.
Fig. 15 is a chart illustrating peaks separated for analysis on the WAXS scattering curve of Sample B.

Description of Embodiments

Hereinafter, a blood sampling needle will be illustrated as a medical needle according to the present invention. However, the present invention is not particularly limited to the blood sampling needle.

Fig. 1 is a perspective view of a blood sampling needle 10. Fig. 2 is a longitudinal cross-sectional view illustrating main parts of the blood sampling needle 10. The blood sampling needle 10 includes a needle body 12, a needle hub 14 (hub), and a protector 16.

As illustrated in Figs. 1 and 2, the needle body 12 is a circular tubular body having a small diameter and a long length. The needle body 12 has a proximal portion 20, or one end in a longitudinal direction, and a distal portion 22, or the other end in the longitudinal direction. The needle body 12 is provided with a lumen 24. The lumen 24 is a blood channel, having a hollow interior extending from the proximal portion 20 to the distal portion 22. The lumen 24 makes the needle body 12 hollow.

The proximal portion 20 of the needle body 12 is inserted into the needle hub 14. Specifically, the needle hub 14 is provided with a through hole 26 extending along an axial direction of the needle hub 14. The through hole 26 in the needle hub 14 extends from a distal end face of a distal end 28 that faces the needle body 12 to a protruding end surface of a protrusion 30. The through hole 26 includes an introducing hole 32, a retaining hole 34, and a communicating hole 36. The introducing hole 32 opens at the distal end face of the distal end 28 in the needle hub 14. The retaining hole 34 is interposed between the introducing hole 32 and the communicating hole 36.

The introducing hole 32 is tapered, increasing in diameter from a proximal end 38 toward the distal end 28 of the needle hub 14. The proximal portion 20 of the needle body 12 is inserted from the introducing hole 32, and then, pushed into the retaining hole 34. The proximal portion 20 of the needle body 12 is joined to the inner wall of the retaining hole 34 with an adhesive involved.

The proximal end 38 of the needle hub 14 is provided with an inserting groove 40. The cylindrical protrusion 30 is disposed on the bottom of the inserting groove 40. The protrusion 30 protrudes toward the proximal end 38 of the needle hub 14. However, the entire protrusion 30 is housed in the inserting groove 40. In other words, the protrusion 30 is not exposed from the inserting groove 40.

A distal end of the tube 42 is inserted into the inserting groove 40. The distal end of the tube 42 is fitted into the protrusion 30. A lumen 44 of the tube 42 is communicated with the lumen 24 of the needle body 12 through the communicating hole 36.

As illustrated in Figs. 1 and 2, the protector 16 is a hollow member. The protector 16 has an inner layer 50 and an outer layer 52. In other words, the protector 16 has a double structure of the inner layer 50 and the outer layer 52. The inner layer 50 is a needle sheath that covers and shields the needle body 12. In the needle body 12, a portion covered by the inner layer 50 is exposed from the distal end 28 of the needle hub 14. In the needle body 12, the portion exposed from the distal end 28 of the needle hub 14 includes the distal portion 22. The outer layer 52 covers the inner layer 50. In other words, the inner layer 50 is housed in the outer layer 52. In this manner, the outer layer 52 serves as an outer sheath.

In the blood sampling needle 10, before the tube 42 is connected to the protrusion 30, a sealing film is applied to the opening of the inserting groove 40. In this state, the blood sampling needle 10 is sterilized with high-pressure steam. During the sterilization, at least the inner layer 50 or the outer layer 52 of the protector 16 is joined to the distal end 28 of the needle hub 14. Accordingly, a space between the protector 16 and the needle hub 14 is sealed in a state where the interior

of the inner layer 50 (housing space for the needle body 12) is kept sterile.

When using the blood sampling needle 10, a user holds the protector 16 with one hand and holds the needle hub 14 with the other hand. Next, the user twists the protector 16 or the needle hub 14. With this operation, the protector 16 and the needle hub 14 are separated from each other. Accordingly, the needle body 12 is exposed. Optionally, the protector 16 is attached to the needle hub 14 to cover the needle body 12. In this case, the protector 16 is detachable from the needle hub 14.

In this configuration, the needle hub 14 includes a first resin composition. The inner layer 50 of the protector 16 includes a second resin composition. The first resin composition and the second resin composition will now be described.

The first resin composition contains a base resin and a plasticizer. In this embodiment, the base resin is polyvinyl chloride (PVC). The plasticizer is a trimellitate. Specific examples of the trimellitate include tributyl trimellitate and tris (2-ethylhexyl) trimellitate. Hereinafter, tris (2-ethylhexyl) trimellitate is also referred to as "TOTM". The following formula is the chemical structural formula of TOTM.

[Chemical Formula 1]

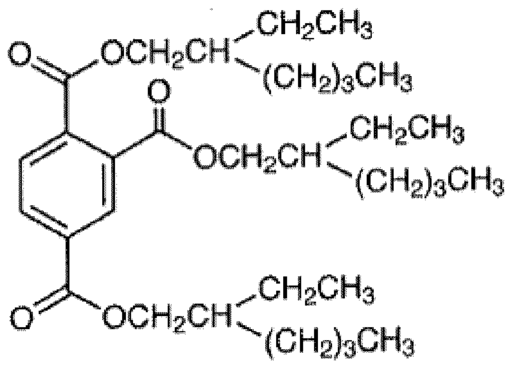

In the first resin composition, the trimellitate accounts for 10 to 20 phr. In other words, the weight of the trimellitate is 10 to 20 when the weight of PVC as the base resin is 100. With less than 10 phr of the trimellitate, the hardness of the needle hub 14 increases. The increased hardness makes it difficult to separate the protector 16 from the needle hub 14. In addition, it is not easy to attach the protector 16 to the needle hub 14 after separating the protector 16 from the needle hub 14. With over 20 phr of the trimellitate, the hardness of the needle hub 14 decreases. The decreased hardness causes concern such as misalignment of the needle body 12 with the needle hub 14.

In other words, setting the proportion of the trimellitate within the range of 10 to 20 phr facilitates separation of the protector 16 from the needle hub 14 and attachment of the protector 16 to the needle hub 14. Furthermore, it is possible to maintain a state where the needle body 12 is firmly retained by the needle hub 14. More preferably, the trimellitate in the first resin composition accounts for 13 to 18 phr.

The second resin composition contains a base resin and a plasticizer. In this embodiment, the base resin is PVC. The plasticizer is a trimellitate. Specific examples of the trimellitate include tributyl trimellitate and TOTM.

In the second resin composition, the trimellitate accounts for 40 to 50 phr. In other words, the weight of the trimellitate is 40 to 50 when the weight of PVC as the base resin is 100. With less than 40 phr of the trimellitate, the hardness of the protector 16 increases. The increased hardness requires large torque when the protector 16 is separated from the needle hub 14. With over 50 phr of the trimellitate, the hardness of the protector 16 decreases. The decreased hardness causes the protector 16 to squash slightly when the protector 16 is held. Furthermore, when the protector 16 is twisted relative to the needle hub 14, a joint between the protector 16 and the needle hub 14 extends. For these reasons, it is not easy to separate the protector 16 from the needle hub 14.

In other words, setting the proportion of the trimellitate within the range of 40 to 50 phr facilitates separation of the protector 16 from the needle hub 14. It is also easy to attach the separated protector 16 to the needle hub 14. More preferably, the trimellitate in the second resin composition accounts for 45 to 47 phr.

In this embodiment, materials of the inner layer 50 and the outer layer 52 are equally the second resin composition. In other words, the inner layer 50 and the outer layer 52 contain the same proportion of the trimellitate. In this case, the protector 16 is easily prepared. Alternatively, the second resin composition of the inner layer 50 and the second resin composition of the outer layer 52 may have different proportions of the trimellitate.

Materials of the inner layer 50 and the outer layer 52 may be different materials. Even in this case, the inner layer 50 is formed using the second resin composition as a material. Preferably, specific examples of the material for the outer layer 52 include polyethylene and polycarbonate.

The blood sampling needle 10 according to this embodiment is basically configured as described above. Next, effects of the blood sampling needle 10 will be described.

Before use, the blood sampling needle 10 is sterilized with high-pressure steam. During the sterilization, at least the inner layer 50 or the outer layer 52 of the protector 16 is joined to the distal end 28 of the needle hub 14.

When using the blood sampling needle 10, a user connects the distal end of the tube 42 to the protrusion 30 of the needle hub 14. Note that a proximal end of the tube 42 is connected to a bag (not illustrated) at this point in time. After that, the user holds the protector 16 and the needle hub 14. Next, the user twists the protector 16 relative to the needle hub 14. With this operation, the joint between the protector 16 and the needle hub 14 is broken. In other words, the protector 16 is separated from the needle hub 14.

Herein, material of the needle hub 14 is the first resin composition described above. Material of the protector 16 is the second resin composition described above. When joining the needle hub 14 including the first resin composition and the protector 16 including the second resin composition, it is possible to separate the protector 16 and the needle hub 14 with ease and with small rotary torque.

Along with the separation of the protector 16 from the needle hub 14, the distal portion 22 of the needle body 12 is exposed from the protector 16. The user inserts the distal portion 22 of the needle body 12 into a patient's blood vessel. After that, blood is collected from the blood vessel through the needle body 12. The blood flows into the bag through the lumen 24 of the needle body 12, the communicating hole 36 of the needle hub 14, and the lumen 44 of the tube 42. After a predetermined amount of blood is collected in the bag, the user draws the distal portion 22 of the needle body 12 from the blood vessel.

Material of the needle hub 14 is the first resin composition. The needle hub 14 including the first resin composition provides an excellent retaining force with respect to the needle body 12. Therefore, when the blood sampling needle 10 is drawn from the blood vessel, the needle body 12 moves in an integrated manner with the needle hub 14.

The protector 16 is put on the needle body 12 of the used blood sampling needle 10. This prevents the needle body 12 from piercing any object. Material of the needle hub 14 is the first resin composition. Material of the protector 16 is the second resin composition. When the protector 16 including the second resin composition is attached to the needle hub 14 including the first resin composition, the attachment is easy. It is also easy to remove the protector 16 from the needle hub 14 after the attachment.

Note that the present invention is defined in independent claim 1. Further, beneficial embodiments are defined in the dependent claims.

For example, the medical needle is not particularly limited to the blood sampling needle 10. Other specific examples of the medical needle include an injection needle and a puncture needle.

## Examples

### [Example 1]

A needle hub 14 was prepared from a first resin composition containing 15 phr of TOTM added to PVC. A proximal portion 20 of a needle body 12 was supported by the needle hub 14. On the other hand, a plurality of kinds of protectors 16 was individually prepared from a second resin composition containing 45 phr, 46 phr, or 47 phr of TOTM added to PVC. After that, each protector 16 was attached to the needle hub 14, followed by sterilization with a high-pressure steam sterilizer. A plurality of kinds of blood sampling needles 10 was obtained through these steps.

Next, each protector 16 was retained by a clamp of a torque gauge. An inspector held the needle hub 14 with one hand and held the torque gauge with the other hand. After that, rotary torque was applied to each protector 16, thereby measuring the rotary torque when each protector 16 was separated from the needle hub 14. Fifty blood sampling needles 10 were measured to calculate average rotary torque.

For comparison, a blood sampling needle according to the related art was prepared. This blood sampling needle is hereinafter referred to as Comparative Example. Material of a needle hub in Comparative Example is a resin composition obtained by adding DEHP to PVC. Material of a protector in Comparative Example is a resin composition obtained by adding DEHP to PVC. As similar to the blood sampling needles in Example, fifty blood sampling needles in Comparative Example were measured to calculate average rotary torque when the protector was separated from the needle hub.

The results are collectively illustrated in a graph in Fig. 3. In Fig. 3, the rotary torque is taken along the ordinate. The unit of the rotary torque is cN·m. Fig. 3 illustrates that the average rotary torque of the blood sampling needle 10 according to this embodiment is smaller than the average rotary torque in Comparative Example.

Although not particularly illustrated, when needle hubs 14 were prepared from a first resin composition obtained by adding 16 phr, 17 phr, or 18 phr of TOTM to PVC, substantially similar results to Fig. 3 were obtained. In other words, the blood sampling needle 10 according to this embodiment makes it easier to separate the protector 16 from the needle hub 14 than the blood sampling needle according to the related art.

[Example 2]

A plurality of kinds of needle hubs 14 was individually prepared from a first resin composition containing 15 phr, 16 phr, 17 phr, or 18 phr of TOTM added to PVC. A proximal portion 20 of a needle body 12 was supported by the needle hub 14. Next, each needle hub 14 was held by a stationary clamp, and the needle body 12 was held by a variable clamp. In this state, the variable clamp was moved in a direction away from the stationary clamp to impart tensile strength to the needle body 12. With this operation, the tensile strength when the needle body 12 was detached from each needle hub 14 was measured. One hundred test samples were measured to calculate average tensile strength. Similarly, one hundred test samples of Comparative Example provided with no protector was measured to calculate average tensile strength.

The results are illustrated in Fig. 4. Note that the unit of the ordinate in Fig. 4 is N. Fig. 4 illustrates that the average tensile strength of the blood sampling needle 10 according to this embodiment is substantially equal to the average tensile strength in Comparative Example. In other words, in this embodiment, the needle hub 14 provides a sufficient retaining force with respect to the needle body 12. Therefore, it is unlikely that the needle body 12 is misaligned with the needle hub 14.

[Example 3]

A plurality of kinds of needle hubs 14 was individually prepared from a first resin composition containing 15 phr, 16 phr, 17 phr, or 18 phr of TOTM added to PVC. A proximal portion 20 of a needle body 12 was supported by the needle hub 14. On the other hand, a protector 16 was prepared from a second resin composition obtained by adding 45 phr of TOTM to PVC. After that, each protector 16 was attached to the needle hub 14, followed by sterilization with a high-pressure steam sterilizer. A plurality of kinds of blood sampling needles 10 was obtained through these steps.

After the protector 16 was separated from each needle hub 14, each needle hub 14 was held by a stationary clamp, and the protector 16 was held by a variable clamp. Next, the variable clamp was moved toward the stationary clamp, and the protector 16 was attached to each needle hub 14. In addition, the variable clamp was moved in a direction away from the stationary clamp, and the protector 16 attached to each needle hub 14 was drawn from each needle hub 14. An attachment/detachment force required for the attachment and the drawing was measured. One hundred blood sampling needles 10 were measured to calculate an average attachment/detachment force. Similarly, one hundred blood sampling needles in Comparative Example were measured to calculate an average attachment/detachment force.

The results are illustrated in Fig. 5. Note that the unit of the ordinate in Fig. 5 is N. Fig. 5 illustrates that the average attachment/detachment force of the blood sampling needle 10 according to this embodiment is substantially equal to the average attachment/detachment force in Comparative Example. Although not particularly illustrated, when protectors 16 were prepared from a second resin composition obtained by adding 46 phr or 47 phr of TOTM to PVC, substantially similar results to Fig. 5 were obtained.

In other words, in this embodiment, it is easy to attach the protector 16 to the needle hub 14 and to detach the protector 16 attached to the needle hub 14 from the needle hub 14.

[Example 4]

A resin composition obtained by adding 45 phr of TOTM to PVC was molded into a cylindrical shape to obtain a molded article. An annular test sample was cut out from the molded article. This is referred to as Sample A. Another test sample having the same shape as that of Sample A was obtained from a resin composition obtained by adding 45 phr of DEHP to PVC. This is referred to as Sample B.

Sample A and Sample B were subjected to small-angle X-ray scattering (SAXS) in a vacuum atmosphere using NANOSTAR available from Bruker AXS. CuK$\alpha$ rays were used as X-rays, and the output was set to 45 kV/120 mA. The camera length was 104.4 cm, and the exposure time was 180 minutes. A SAXS scattering curve is illustrated in Fig. 6 where the wavelength is taken along the abscissa and the intensity is taken along the ordinate. Furthermore, the SAXS scattering curve of Sample A was subjected to curve fitting. The result is illustrated in Fig. 7.

Fig. 8 schematically illustrates an ideal crystal. In the ideal crystal, atoms are arranged regularly. Fig. 9 schematically illustrates amorphousness. The amorphousness has irregularly arranged atoms. As illustrated in Fig. 10A, Sample A has a structure where crystalline regions and an amorphous region are mixed, but it is inferred from Figs. 6 and 7 that the amorphous region is less disordered and that the crystalline regions and the amorphous region are clearly separated. In other words, as illustrated in Fig. 10B, Sample A has clear boundaries between the crystalline regions and the amorphous region.

In a similar manner, the SAXS scattering curve of Sample B was also subjected to curve fitting. The result is illustrated in Fig. 11. As illustrated in Fig. 12A, Sample B has a structure where the degree of disorder of an amorphous region is larger than that of Sample A, and it is inferred from Figs. 6 and 11 that crystalline regions and the amorphous region are not clearly separated. In other words, as illustrated in Fig. 12B, between the crystalline regions and the

amorphous region, Sample B has transition regions where the crystalline regions and the amorphous region are mixed. Therefore, boundaries between the crystalline regions and the amorphous region are unclear.

In the structure illustrated in Fig. 10B, stresses are concentrated on interfaces between the crystalline regions and the amorphous region. In contrast, in the structure illustrated in Fig. 12B, it is relatively difficult to cause stress concentration on interfaces between the crystalline regions and the amorphous region. This is considered to be the reason that the protector 16 is easily separated from the needle hub 14 in the embodiment described above.

[Example 5]

Wide angle X-ray scattering (WAXS) was performed on each of Sample A and Sample B in a similar manner to the aforementioned Example except that the camera length was 4.55 cm and the exposure time was 60 minutes. A WAXS scattering curve is illustrated in Fig. 13 where the wavelength is taken along the abscissa and the intensity is taken along the ordinate.

Peaks in the WAXS scattering curve of Sample A were separated for analysis. The result is illustrated in Fig. 14. Dash-dotted lines in Fig. 14 indicate peaks derived from the crystalline regions. A dashed line in Fig. 14 indicates a peak derived from the amorphous region. A crystallinity index is calculated from the following Formula based on a total area of peaks derived from the crystalline regions and an area of a peak derived from the amorphous region.

[Math. 1]

$$\text{CRYSTALLINITY INDEX [\%]} = \frac{\text{TOTAL AREA OF PEAKS DERIVED FROM CRYSTALLINE REGIONS}}{\text{TOTAL AREA OF PEAKS DERIVED FROM CRYSTALLINE REGIONS + AREA OF PEAK DERIVED FROM AMORPHOUS REGION}} \times 100$$

The crystallinity index of Sample A calculated from the above Formula was 57%.

Peaks in the WAXS scattering curve of Sample B were also separated for analysis. The result is illustrated in Fig. 15. Like Fig. 14, dash-dotted lines indicate peaks derived from the crystalline regions, and a thin dashed line indicates a peak derived from the amorphous region. The crystallinity index of Sample B calculated from the above Formula was 59%.

The crystallinity index of Sample A is smaller than the crystallinity index of Sample B. However, in Sample A, as described above, there are clear interfaces between the crystalline regions and the amorphous region. Based on this result, it is inferred that the blood sampling needle 10 according to this embodiment enables separation of the protector 16 from the needle hub 14 with small rotary torque.

## Claims

1. A medical needle (10) comprising: a needle body (12); a hub (14) that supports a proximal portion (20) of the needle body (12) in a longitudinal direction; and a protector (16) that protects the needle body (12), wherein

   the protector (16) has a needle sheath that covers a portion of the needle body (12) exposed from the hub (14),
   the needle sheath is separably joined to the hub (14),
   the hub (14) includes a first resin composition and the needle sheath includes a second resin composition,
   **characterized in that**
   the first resin composition contains polyvinyl chloride and a trimellitate which accounts for 10 to 20 phr, and the second resin composition contains polyvinyl chloride and a trimellitate which accounts for 40 to 50 phr.

2. The medical needle (10) according to claim 1, wherein the trimellitate in the first resin composition accounts for 13 to 18 phr, and the trimellitate in the second resin composition accounts for 45 to 47 phr.

3. The medical needle (10) according to claim 1 or 2, wherein the trimellitate is tris (2-ethylhexyl) trimellitate.

4. The medical needle (10) according to any one of claims 1 to 3, wherein the protector (16) has an outer sheath that covers the needle sheath.

5. The medical needle (10) according to claim 4, wherein material of the outer sheath is the second resin composition that is same as material of the needle sheath.

## Patentansprüche

1. Medizinische Nadel (10) umfassend: einen Nadelkörper (12); einen Aufsatz (14), der einen proximalen Abschnitt (20) des Nadelkörpers (12) in Längsrichtung trägt; und eine Schutzvorrichtung (16), die den Nadelkörper (12) schützt, wobei

   die Schutzvorrichtung (16) eine Nadelhülle aufweist, die einen Teil des Nadelkörpers (12) bedeckt, der aus dem Aufsatz (14) herausragt,
   die Nadelhülle lösbar mit dem Aufsatz (14) verbunden ist,
   der Aufsatz (14) eine erste Harzzusammensetzung beinhaltet und die Nadelhülle eine zweite Harzzusammensetzung beinhaltet,
   **dadurch gekennzeichnet, dass**
   die erste Harzzusammensetzung Polyvinylchlorid und ein Trimellitat enthält, das 10 bis 20 phr ausmacht, und die zweite Harzzusammensetzung Polyvinylchlorid und ein Trimellitat enthält, das 40 bis 50 phr ausmacht.

2. Medizinische Nadel (10) gemäß Anspruch 1, wobei das Trimellitat in der ersten Harzzusammensetzung 13 bis 18 phr ausmacht und das Trimellitat in der zweiten Harzzusammensetzung 45 bis 47 phr ausmacht.

3. Medizinische Nadel (10) gemäß Anspruch 1 oder 2, wobei das Trimellitat Tris(2-ethylhexyl)trimellitat ist.

4. Medizinische Nadel (10) gemäß einem der Ansprüche 1 bis 3, wobei die Schutzvorrichtung (16) eine Außenhülle aufweist, die die Nadelhülle bedeckt.

5. Medizinische Nadel (10) gemäß Anspruch 4, wobei das Material der Außenhülle die zweite Harzzusammensetzung ist, die mit dem Material der Nadelhülle identisch ist.

## Revendications

1. Aiguille médicale (10) comprenant : un corps d'aiguille (12) ; un embout (14) qui supporte une partie proximale (20) du corps d'aiguille (12) dans une direction longitudinale ; et un protecteur (16) qui protège le corps d'aiguille (12), dans laquelle

   le protecteur (16) a une gaine d'aiguille qui recouvre une partie du corps d'aiguille (12) exposée à partir du embout (14),
   la gaine d'aiguille est reliée de manière séparable à l'embout (14),
   l'embout (14) comporte une première composition de résine et la gaine d'aiguille comporte une deuxième composition de résine,
   **caractérisé en ce que**
   la première composition de résine contient du chlorure de polyvinyle et un trimellitate qui représente 10 à 20 phr, et
   la deuxième composition de résine contient du chlorure de polyvinyle et un trimellitate qui représente 40 à 50 phr.

2. Aiguille médicale (10) selon la revendication 1, dans laquelle le trimellitate dans la première composition de résine représente 13 à 18 phr, et le trimellitate dans la deuxième composition de résine représente 45 à 47 phr.

3. Aiguille médicale (10) selon la revendication 1 ou 2, dans laquelle le trimellitate est du tris (2-éthylhexyl) trimellitate.

4. Aiguille médicale (10) selon l'une quelconque des revendications 1 à 3, dans laquelle le protecteur (16) a une gaine extérieure qui recouvre la gaine de l'aiguille.

5. Aiguille médicale (10) selon la revendication 4, dans laquelle le matériau de la gaine extérieure est la deuxième composition de résine qui est identique au matériau de la gaine de l'aiguille.

FIG. 1

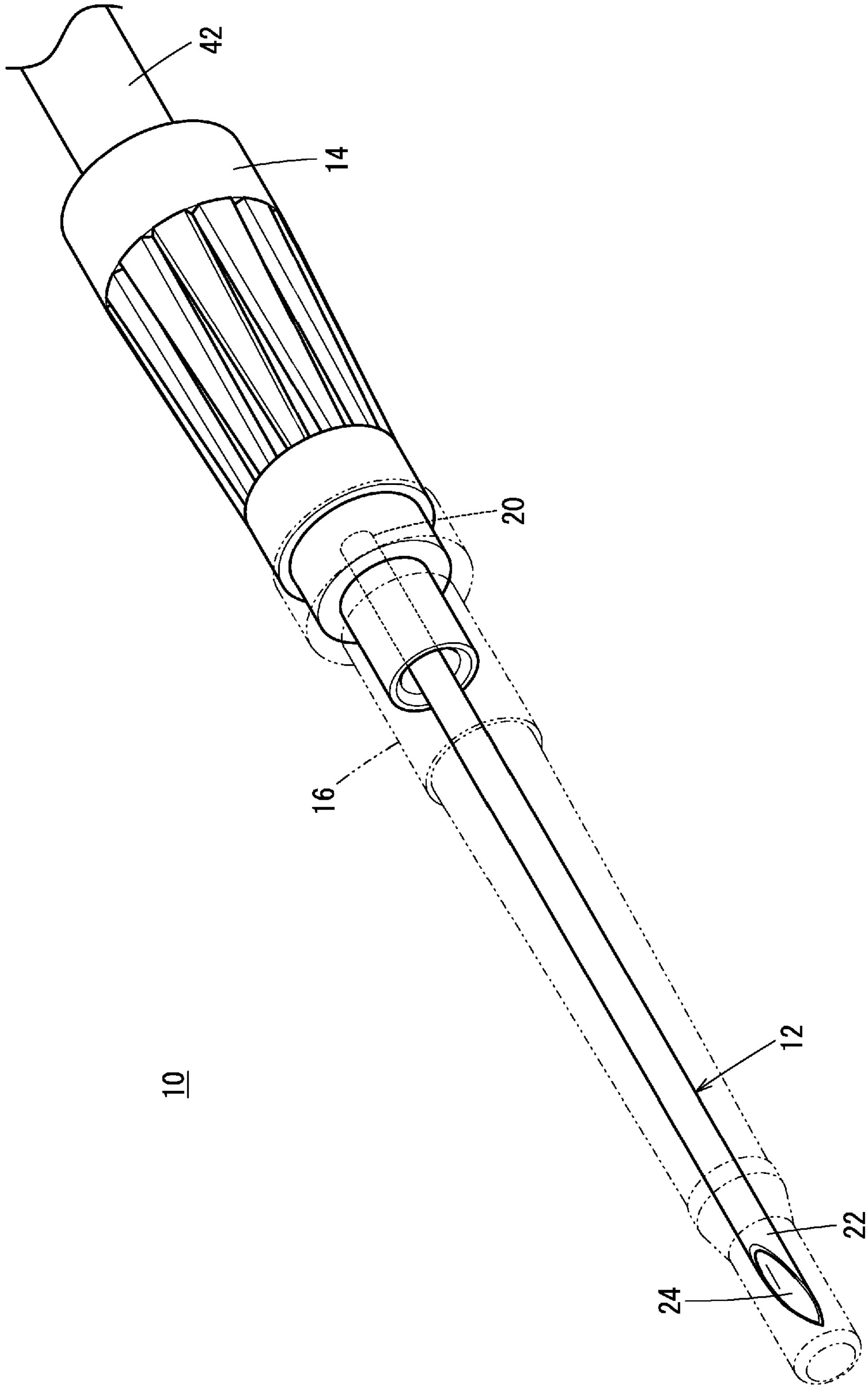

FIG. 2

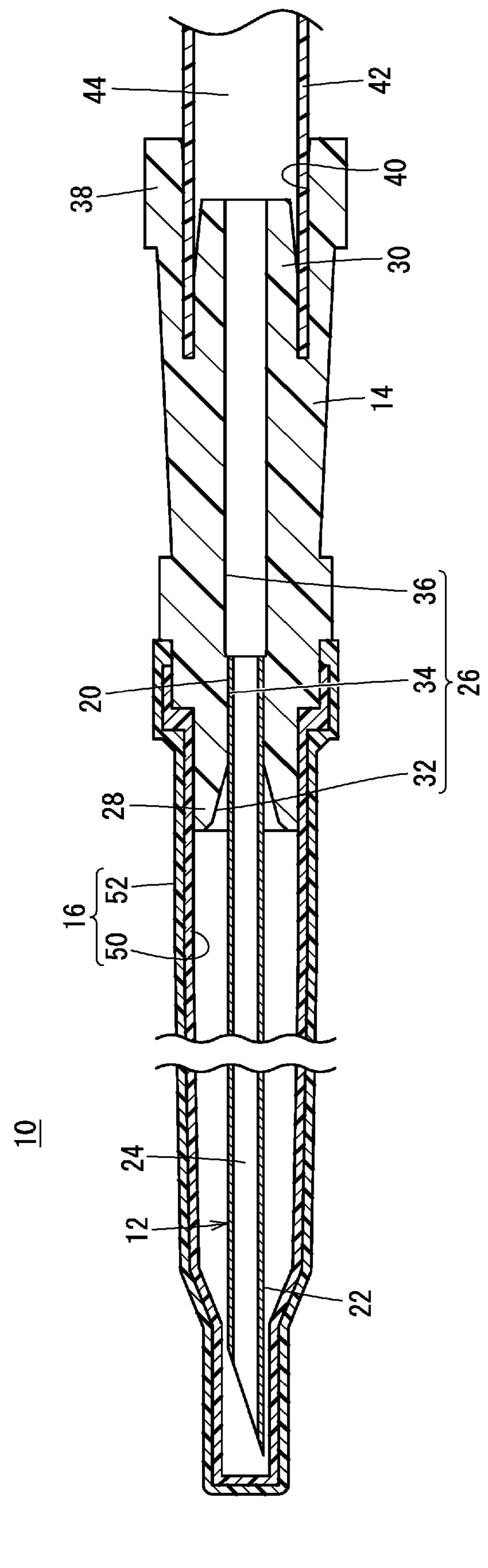

FIG. 3

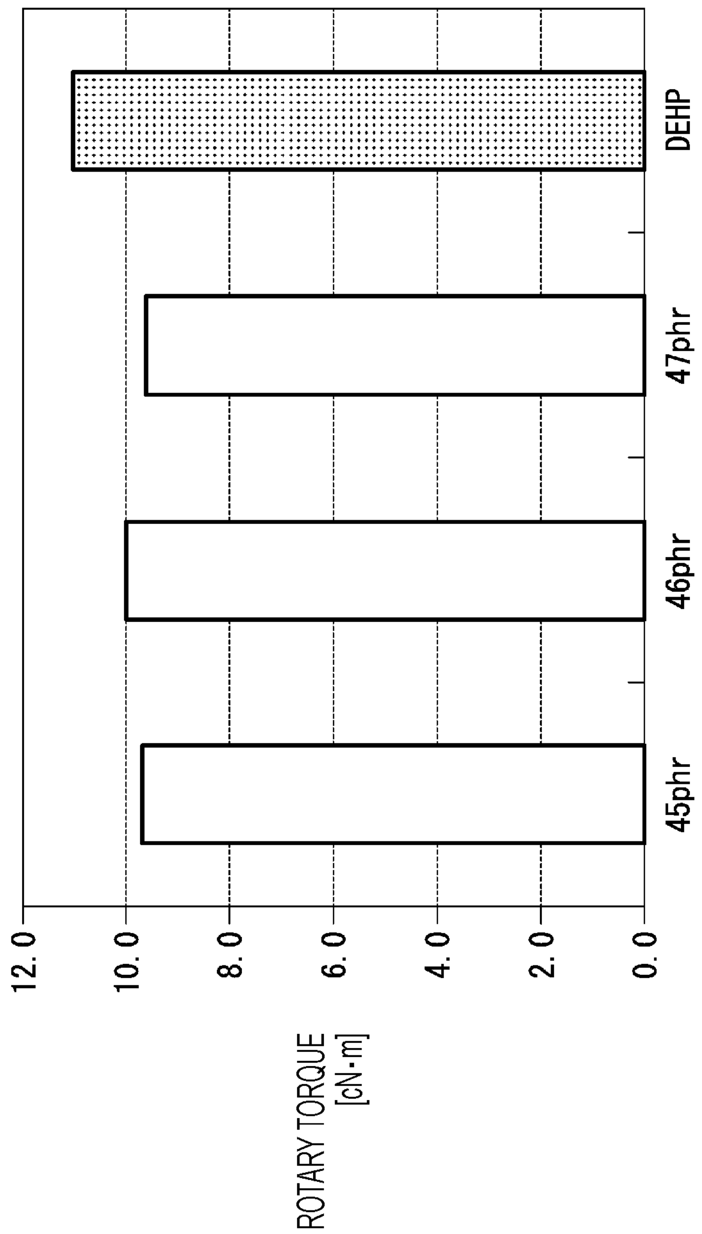
ROTARY TORQUE
[cN·m]
12.0
10.0
8.0
6.0
4.0
2.0
0.0
45phr
46phr
47phr
DEHP

FIG. 4

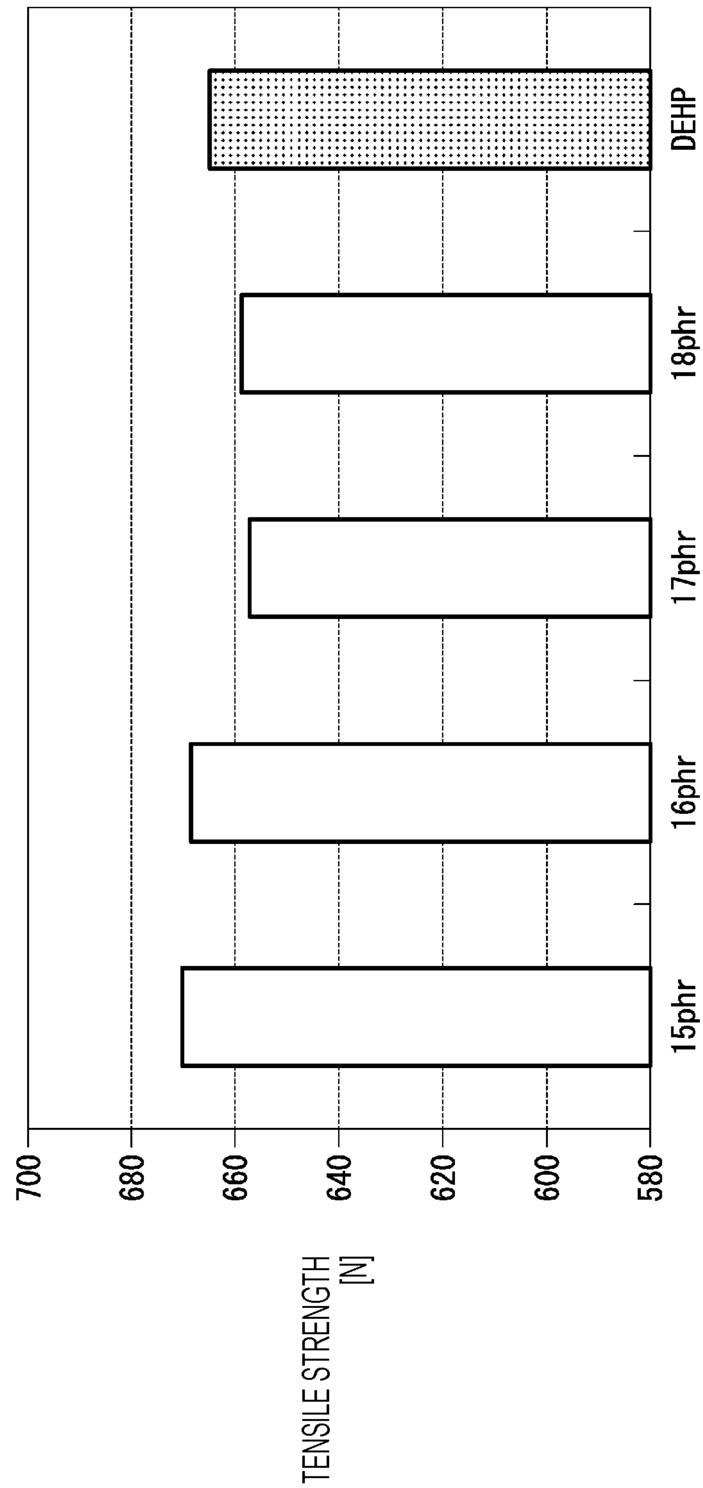
TENSILE STRENGTH
[N]
700
680
660
640
620
600
580
15phr
16phr
17phr
18phr
DEHP

FIG. 5

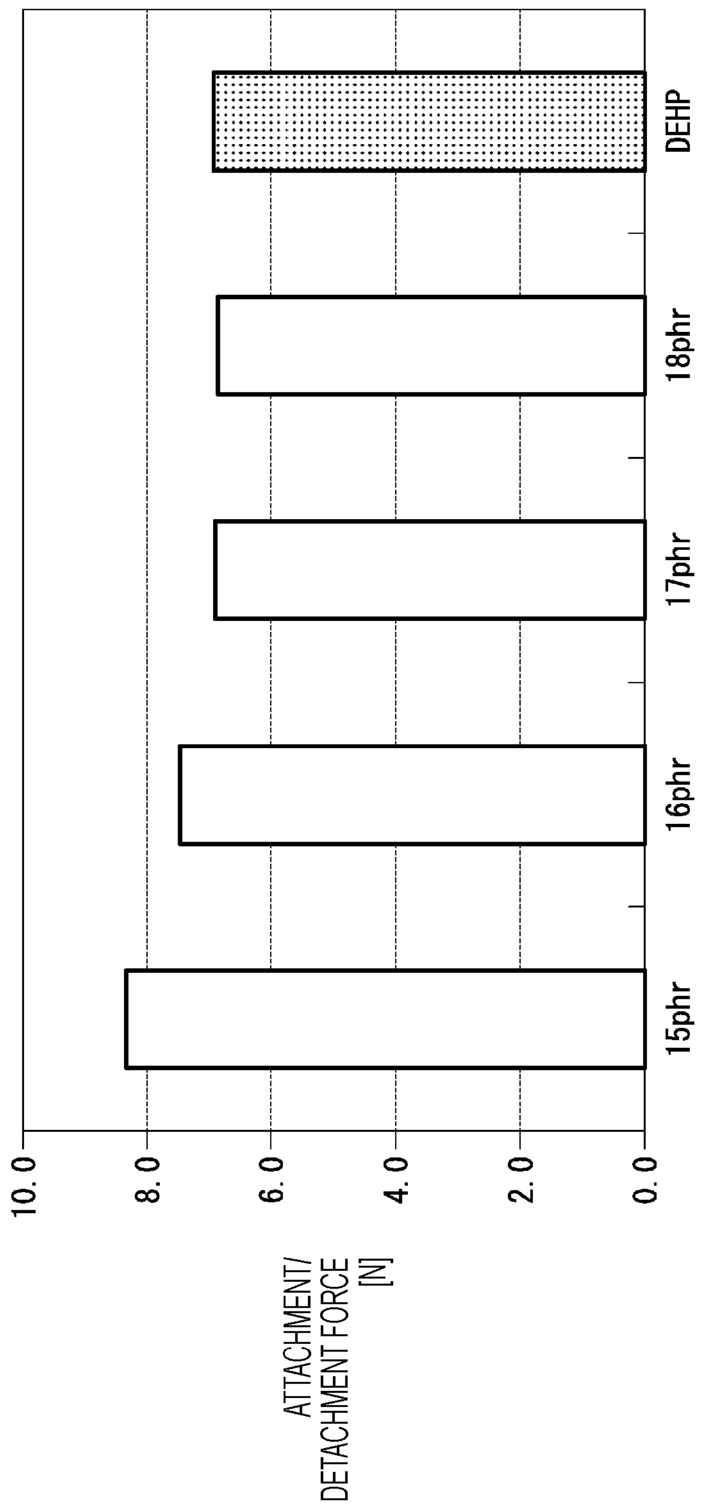
ATTACHMENT/
DETACHMENT FORCE
[N]
10.0
8.0
6.0
4.0
2.0
0.0
15phr
16phr
17phr
18phr
DEHP

FIG. 6

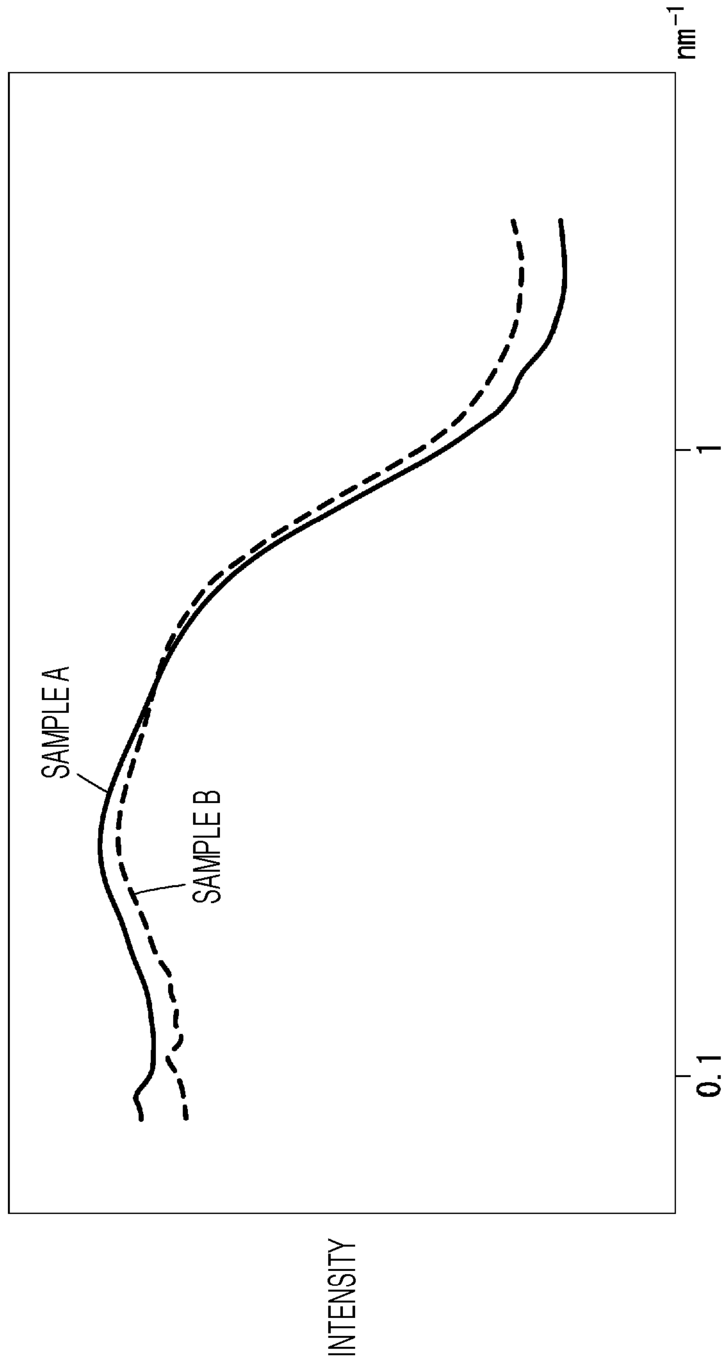

FIG. 7

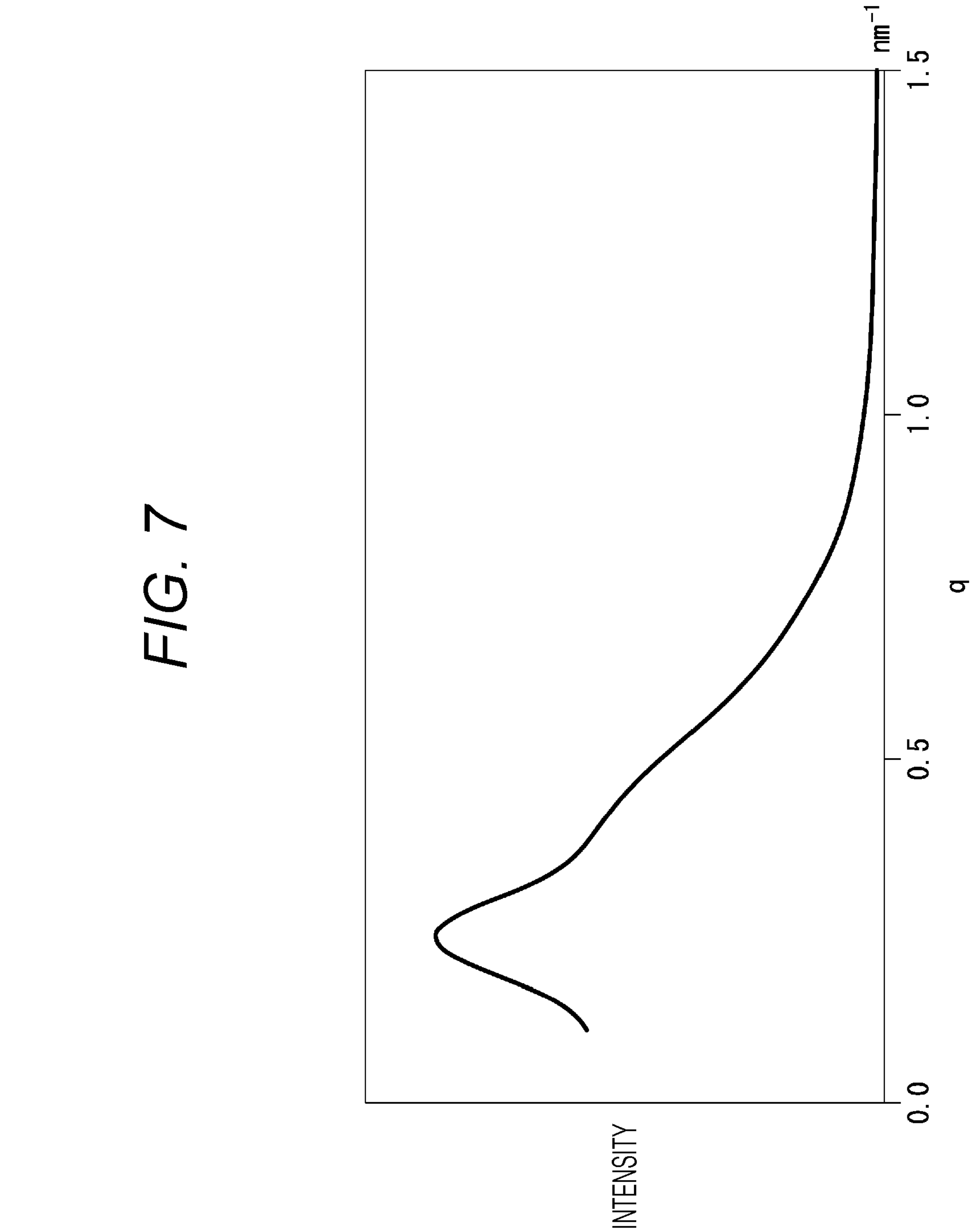

*FIG. 8*

IDEAL CRYSTAL

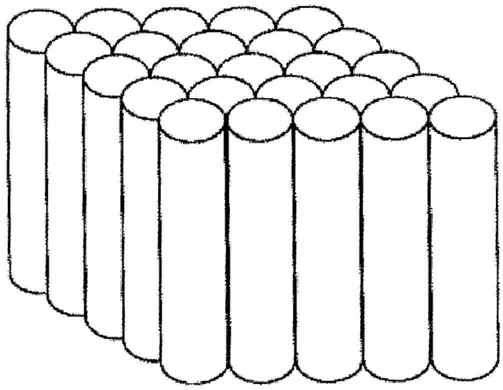

*FIG. 9*

AMORPHOUSNESS

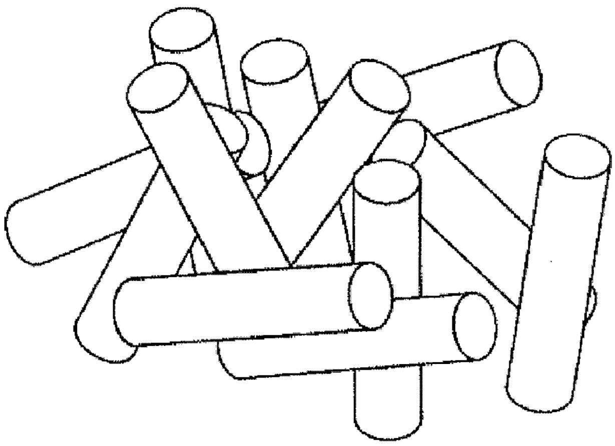

FIG. 10A

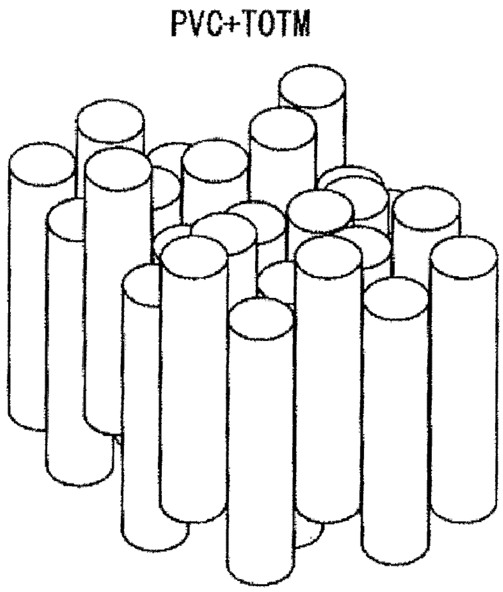


FIG. 10B

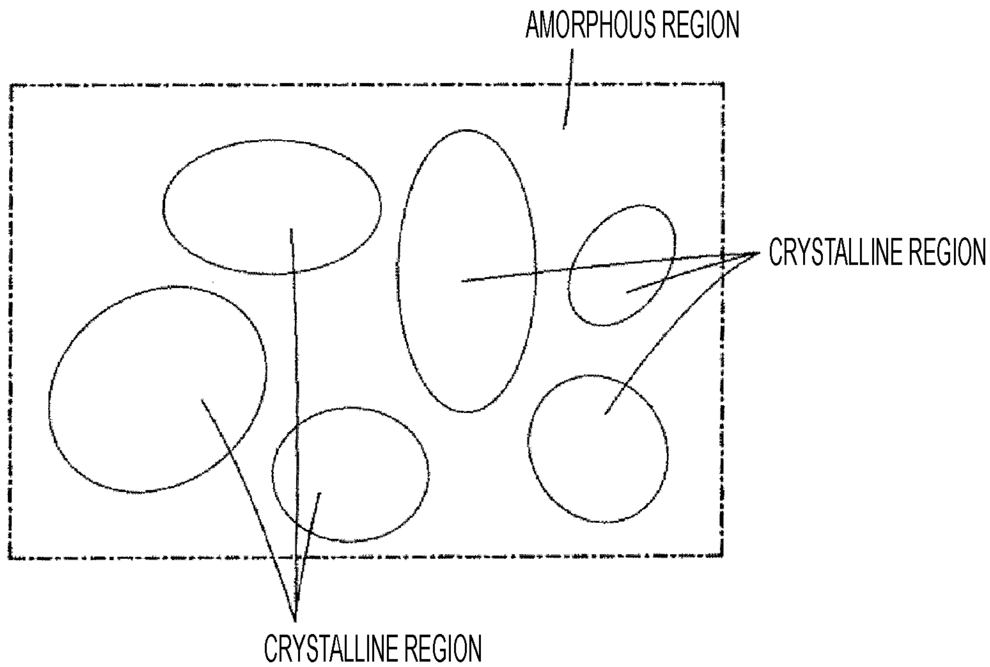

FIG. 11

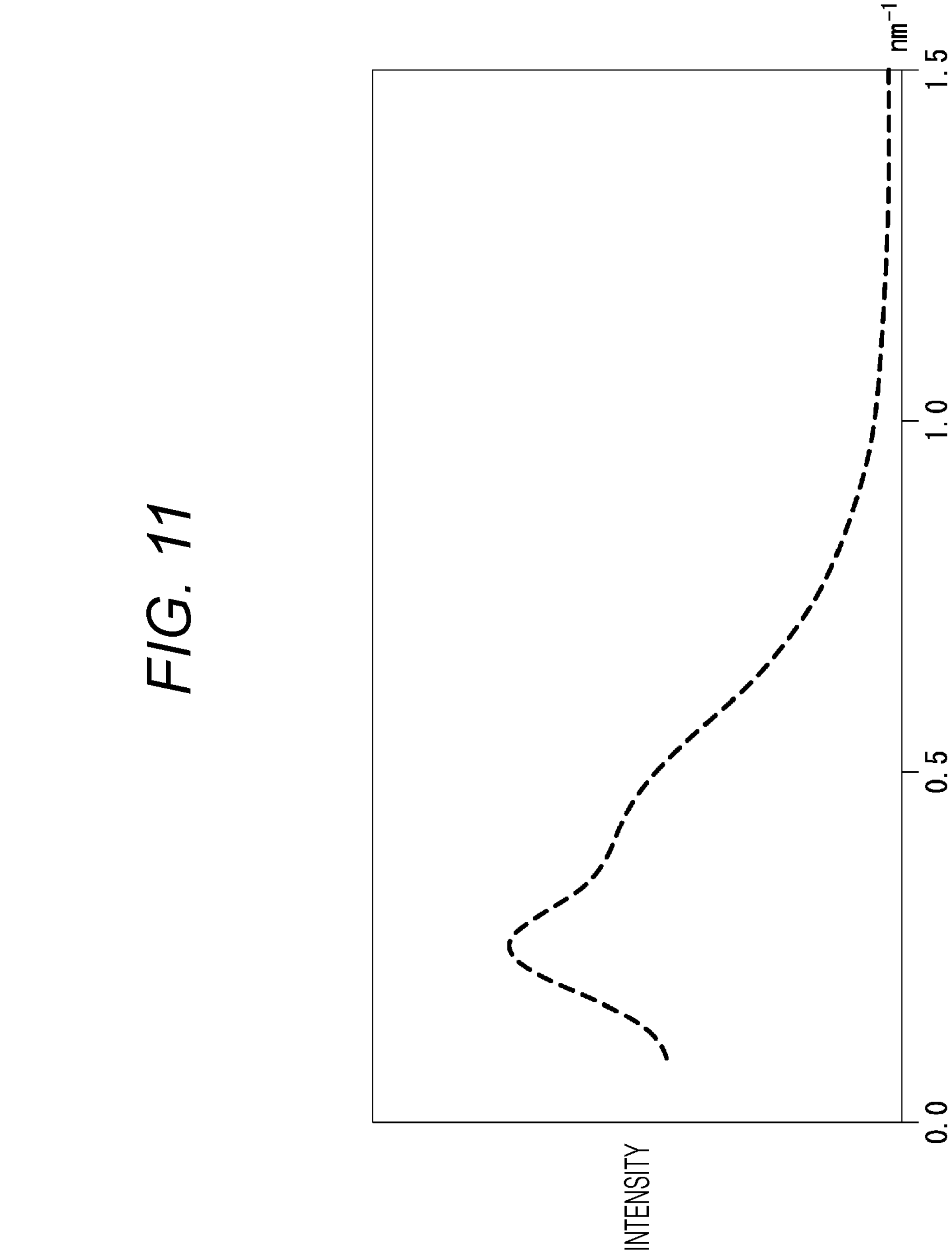

FIG. 12A

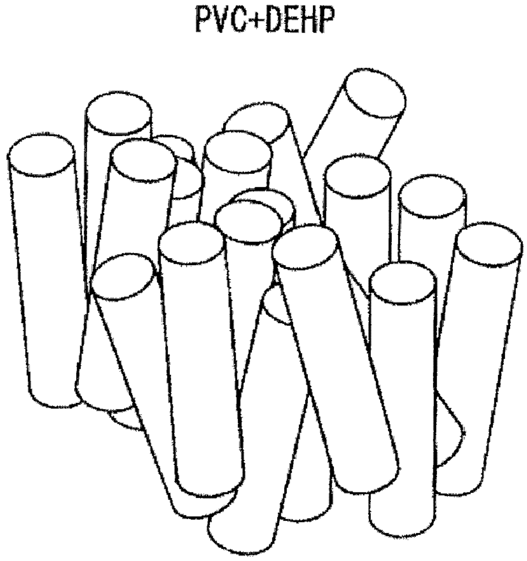


FIG. 12B

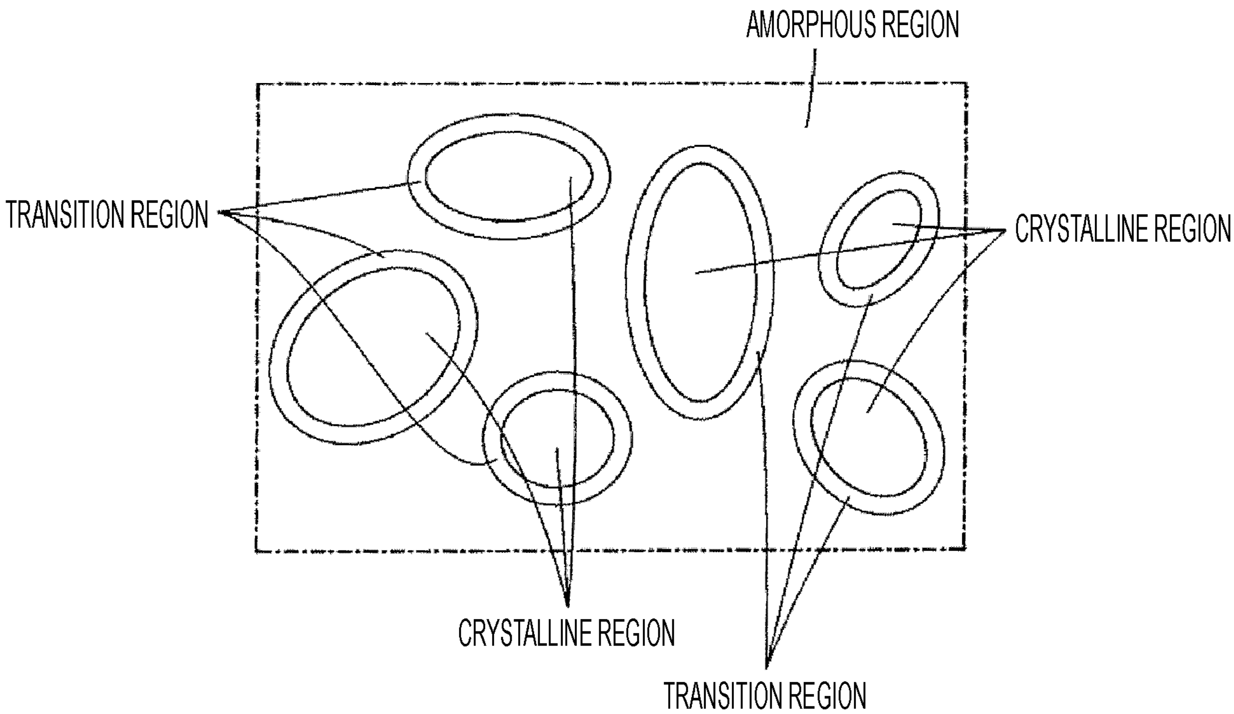

FIG. 13

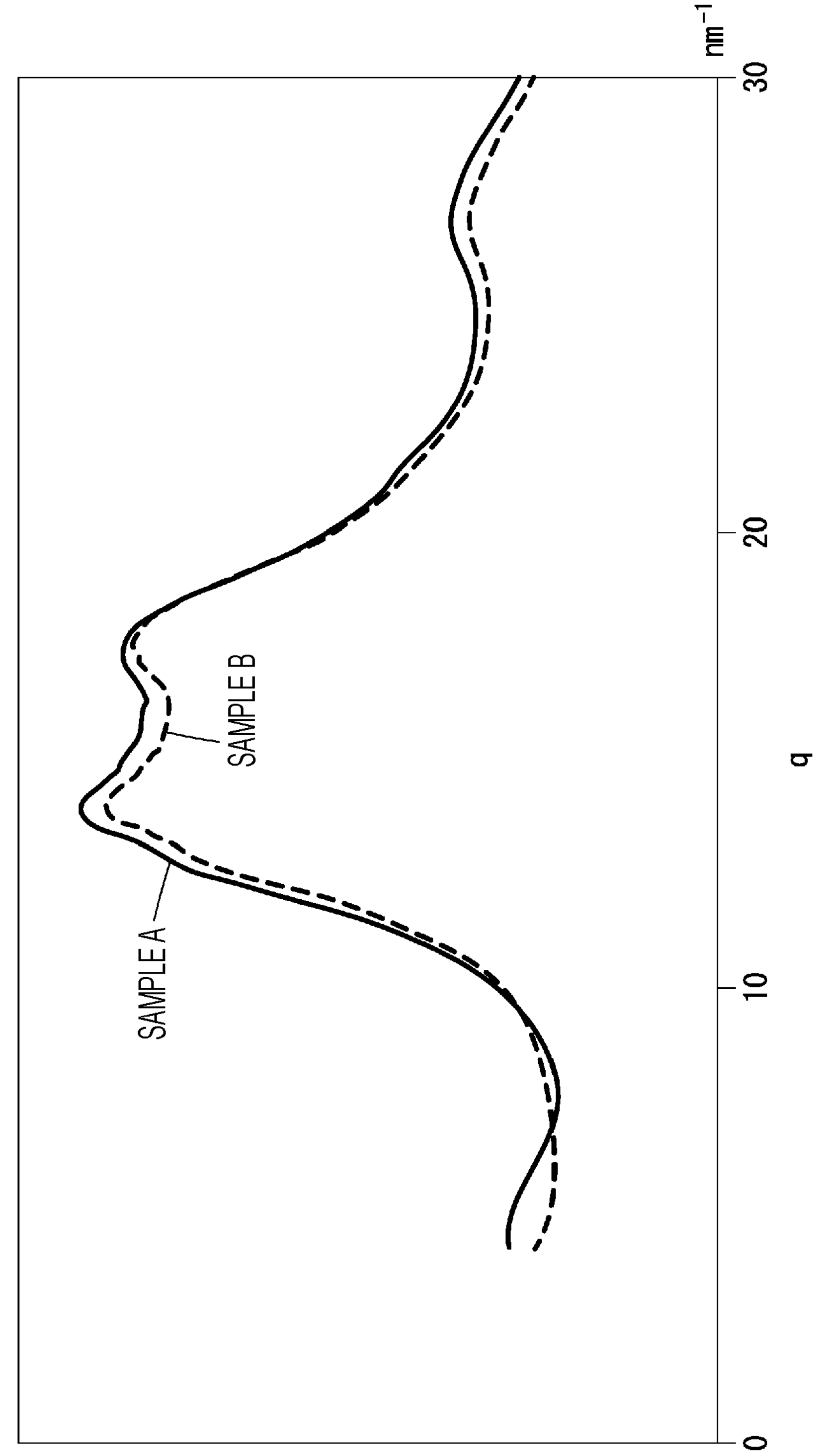

FIG. 14

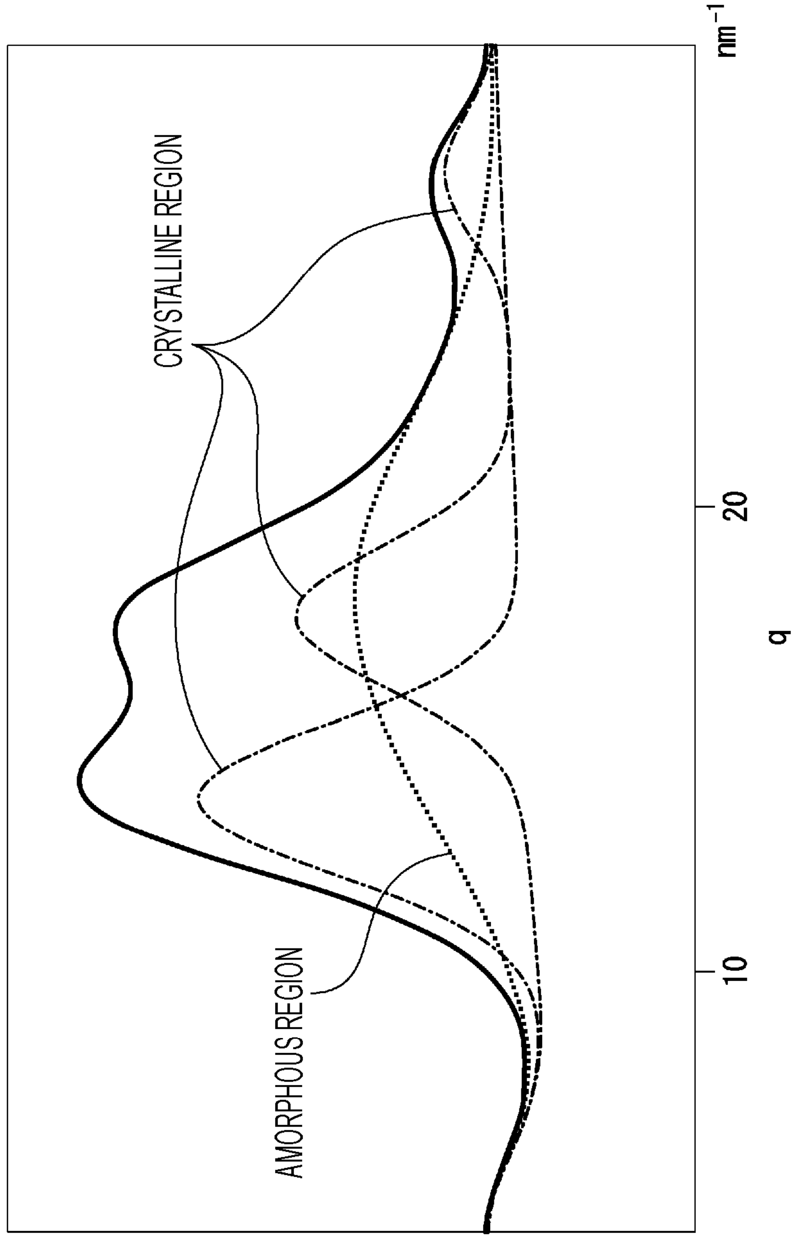

FIG. 15

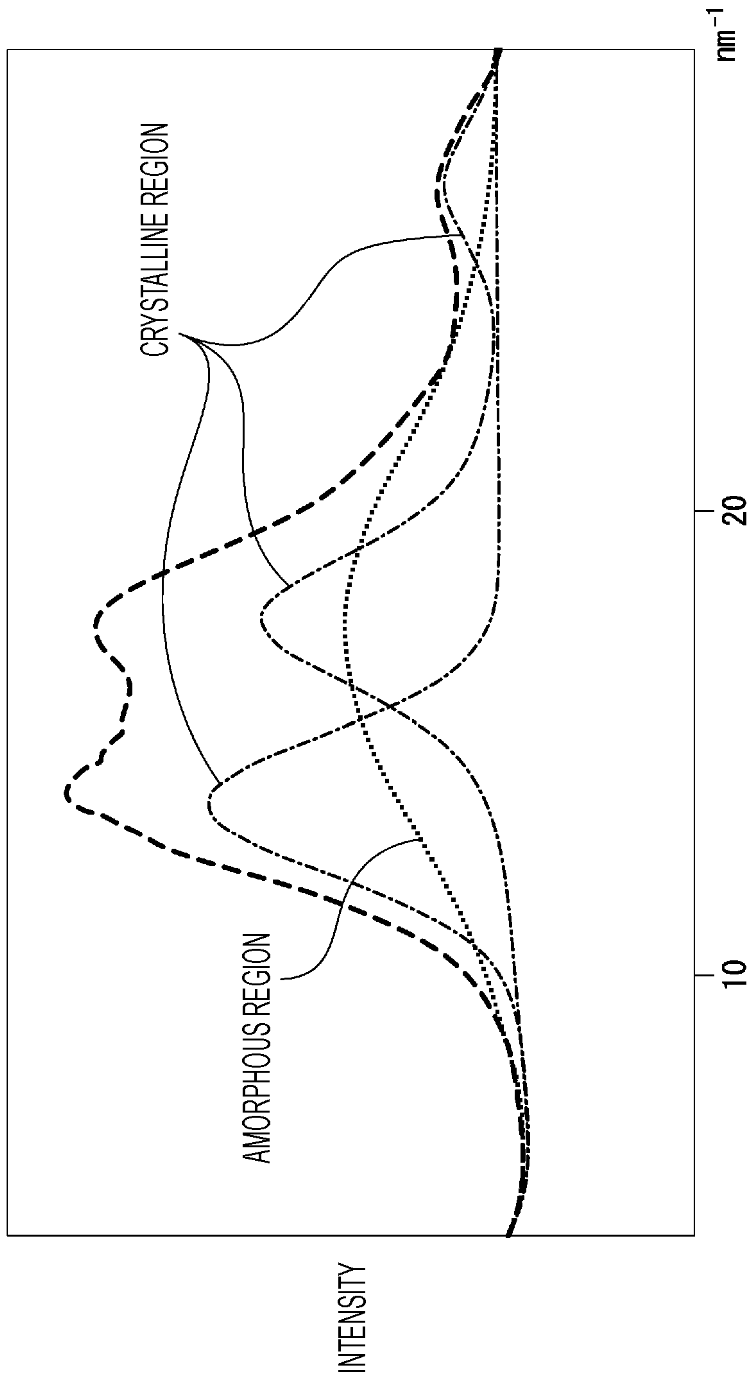

## REFERENCES CITED IN THE DESCRIPTION

*This list of references cited by the applicant is for the reader’s convenience only. It does not form part of the European patent document. Even though great care has been taken in compiling the references, errors or omissions cannot be excluded and the EPO disclaims all liability in this regard.*

### Patent documents cited in the description


- JP 1017381 B